# EUROPEAN PATENT SPECIFICATION

(11) **EP 2 836 504 B1**
(45) Date of publication and mention of the grant of the patent: **14.11.2018**
(21) Application number: 13775393.5
(22) Date of filing: 08.04.2013
(51) Int. Cl.: B01D 15/36, B01D 15/16, C07K 14/505, C07K 1/20

(54) **SINGLE STEP FRACTIONATION METHOD**
EINSTUFIGES FRAKTIONIERUNGSVERFAHREN
PROCÉDÉ DE FRACTIONNEMENT EN UNE SEULE ÉTAPE

(30) Priority: 10.04.2012 IN 1431CH2012
(43) Date of publication of application: 18.02.2015
(73) Proprietor: Dr. Reddy's Laboratories Limited, Qutubullapur, RR District 500 090 (IN)
(72) Inventor: PATRA, Ashish K, Old Town Bhubaneswar 751002 (IN); YETURU, Venkata Ramireddy, Hyderabad 500043 (IN); JACOB, Jaby, Kottayam 686513 (IN)
(74) Representative: Bates, Philip Ian
(86) International application number: PCT/IB2013/052785
(87) International publication number: WO 2013/153497

(56) References cited:
- EP-A1- 1 428 878
- EP-A1- 2 433 952
- EP-A2- 0 428 267
- US-A- 5 981 716
- GOKANA A ET AL: "Chromatographic separation of recombinant human erythropoietin isoforms", JOURNAL OF CHROMATOGRAPHY, ELSEVIER SCIENCE PUBLISHERS B.V, NL, vol. 791, no. 1-2, 12 December 1997 (1997-12-12), pages 109-118, XP004107601, ISSN: 0021-9673, DOI: 10.1016/S0021-9673(97)00766-8
- G, PAUL K. ET AL.: 'Separation of protein mixtures using pH-gradient cation-exchange chromatography.' JOURNAL OF CHROMATOGRAPHY A vol. 1216, 09 January 2009, pages 1372 - 1376, XP025909454
- BARZAGHI, BARBARA. ET AL.: 'Isoelectric protein purification in segmented immobilized pH gradients. Effect of salts on rate of contaminants' removal.' JOURNAL OF BIOCHEMICAL AND BIOPHYSICAL METHODS vol. 15, 1987, pages 177 - 188, XP024355185
- AHRER, KARIN. ET AL.: 'Chromatographic and electrophoretic characterization of protein variants.' JOURNAL OF CHROMATOGRAPHY B vol. 841, 26 July 2006, pages 110 - 122, XP027981922
- PAN, TINGQI. ET AL.: 'Analysis of glycosylation of erythropoietin by isoelectric focusing electrophoresis.' CLIN J BIOLOGICALS. vol. 25, no. 1, January 2012, pages 115 - 117, 122, XP008174898

## Description

### BACKGROUND

Aspects of the present invention relate to a single step fractionation method of highly sialylated variant/s of darbepoetin wherein the method comprises use of a strong anion exchange chromatography support and a pH gradient for elution of the said variant/s.

Darbepoetin (US7217689) is an erythropoiesis stimulating protein. Production of recombinant darbepoetin typically leads to accumulation of heterogeneously sialylated variants of the molecule. A direct correlation between the extent of sialylation and serum half-life of darbepoetin has been established (Egirie et. al Oncology, Vol. 16, 2002, 13-22). Darbepoetin composition comprising higher sialylated variants exhibit enhanced *in vivo* half-life and therefore efficacy, as compared to low sialylated variants. Hence, it is important to fractionate and enrich for higher sialylated variants of the protein.

Different chromatography based methods have been described for separation and enrichment of appropriate darbepoetin variants, in particular highly sialylated variants. However, a significant drawback in the methods described in the prior art are the multistep nature of the fractionation methods.

EP1428878 discloses a method of purifying isoforms of erythropoietin by using at least two anion exchange chromatographic steps separated by one or more chromatographic steps distinct from anion exchange mode. It additionally suggests use of at least one acidic wash step in the anion exchange steps to remove low sialylated forms of the protein.

US7012130 & EP1127063 discloses a multi step method of purifying recombinant human erythropoietin from culture supernatant, by hydrophobic
interaction chromatography, anionic exchange chromatography, cationic exchange chromatography; and molecular exclusion chromatography.

US20110098452 describes a multi step method for purifying low pl isoforms of darbepoetin utilizing at least one cation exchange chromatography in flow through mode, and additional chromatographic steps, which could be anion exchange or mixed mode chromatography.

EP 0428267 A2 discloses in example 5 the purification of erythropoietin isoforms by strong anion-exchange chromatography, followed by reversed-phase chromatography and gel filtration chromatography. Even though, several techniques are described in the art, they are generally multi step fractionation methods that result in low yields, and are generally cumbersome in nature. Therefore, there is a need for development of simpler fractionation methods that require fewer steps for enrichment of highly sialylated variants of a protein.

### SUMMARY

According to the invention there is provided a method for fractionation of highly sialylated variants of darbepoetin comprising:
a) loading a clarified cell culture broth comprising a mixture of differentially sialylated darbepoetin on to a strong anion exchange resin, and
b) eluting the bound protein using a pH gradient at a constant salt concentration, wherein the eluate is enriched in highly sialylated variants of the protein.

### BRIEF DESCRIPTION OF THE DRAWINGS

Fig. 1 Illustration of darbepoetin variant fractionation using a pH gradient at constant salt concentration as described in Example 1.
Fig. 2 Magnified illustration of Region II described in Fig. 1.
Fig. 3 Magnified illustration of Region I described in Fig. 1.
Fig. 4 Isoelectric focusing of fractionated highly sialylated variants as described in Example 1
Fig. 5 Western Blot of Isoelectric focused gel described in Fig. 4 with polyclonal anti-darbepoetin antibody.

### DETAILED DESCRIPTION

According to the invention there is provided a method for fractionation of highly sialylated variants of darbepoetin comprising:
a) loading a clarified cell culture broth comprising a mixture of differentially sialylated darbepoetin on to a strong anion exchange resin, and
b) eluting the bound protein using a pH gradient at a constant salt concentration, wherein the eluate is enriched in highly sialylated variants of the protein.

In a further embodiment the ion exchange chromatography is devoid of any in process wash step.

The anion exchange resin may be pre-equilibrated with a buffer comprising 90 mM sodium chloride. The pH of the equilibration buffer may be between the isoelectric point of the highly sialylated variant of interest and neutral pH.

The conductivity of the elution buffer may be equal to that of the equilibration buffer.

The pH gradient may be established by mixing neutral and acidic pH buffer at a predefined rate such that a linear gradient between near neutral (about 7.5) and acidic (about 2.0) is established.

The term 'highly sialylated variant/s' or 'highly sialylated protein' in the context of the present invention refers to a protein, which contains at least about 18 or more sialic acid moieties attached to the protein.

### EXAMPLE 1

### Sample preparation:

Expression of the darbepoetin was accomplished as described in US 2011/0098452. Harvested cell culture was clarified by centrifugation to obtain CCCB (clarified cell culture broth).

### Instrumentation and Blank

Water Alliance HPLC system housing a strong anion exchange support was used. The differentially eluted variants detected using a PDA detector by measuring UV absorbance at 280nm.
The HPLC profiles for the test samples were analyzed by integrating peaks after subtracting respective buffer blanks.

### Single step fractionation by strong anion exchange chromatography:

ProPac® SAX-10 (Dionex) analytical column with a column volume of about 3ml was pre-equilibrated with 10 mM phosphate, 90 mM NaCl buffer (pH 7.3), followed by loading neat untreated clarified cell culture broth obtained post cell culture on to it. An equilibration salt concentration of 90mM sodium chloride minimized binding of low sialylated variants of the protein to the column. A linear pH gradient was established by mixing the acidic buffer with the neutral buffer (Table 1) at a rate of about 5% percent per minute (Table 2), and flow rate of about 1ml/minute was maintained. Salt concentration of 90mM sodium chloride, equivalent to that of the equilibration buffer was maintained in the acidic and neutral buffer.

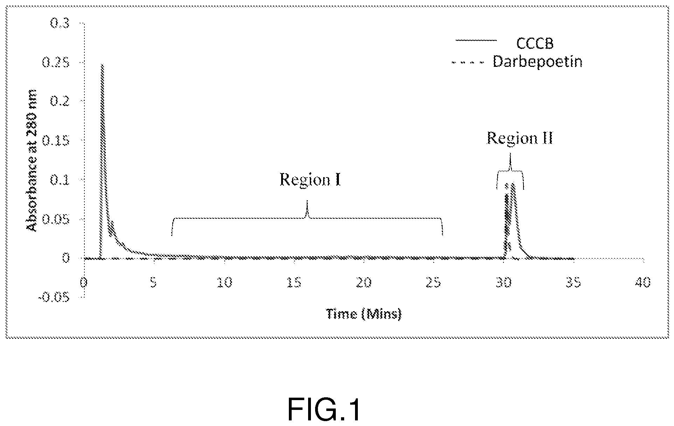

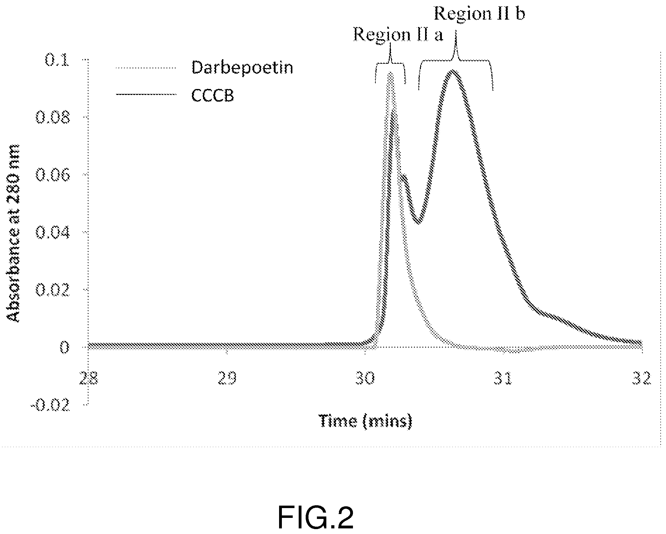

Darbepoetin containing about 18-22 sialylic acid moieties per molecule was used as a control and run under identical conditions. Figure 1 and Figure 2 (magnification of total Region II of Figure 1) illustrates the chromatogram demonstrating concurrent elution of darbepoetin (grey line) with Region IIA corresponding to the highly sialylated variants of darbepoetin from clarified cell culture broth (dark line) at about 30min under given conditions. The pH of the elution buffer was altered from about 7.3 to about 2.5. Elution of highly sialylated variants was obtained at a pH of around 2.5. Region IIB was determined to be non-proteinaceous.

**Table 1: Buffers used for fractionation of sialylated protein**

| S.No | Buffer | Composition |
|---|---|---|
| 1 | Neutral Buffer | 10mM phosphate, 90mM NaCl, pH 7.3 |
| 2 | Acidic Buffer | 5mM phosphoric acid, 90mM NaCl, pH ∼2.5 |

**Table 2: Rate of mixing of neutral and acidic buffers of Table 1**

| **Time (min)** | **Flow rate (mL/min)** | **Mobile phase** | |
|---|---|---|---|
| | | Neutral Buffer % | Acidic Buffer % |
| 0-4 | 1.0 | 100 | 0 |
| 4-24 | 1.0 | 0 | 100 |
| 24-28 | 1.0 | 0 | 100 |
| 28-29 | 1.0 | 100 | 0 |
| 29-35 | 1.0 | 100 | 0 |

A linear pH gradient was attained by mixing at the rate of 5% per minute of the acidic buffer to the neutral buffer. The salt concentration was maintained constant at 90mM sodium chloride.

A correlation between the rate of gradient formation, flow rate and expected elution time of the desired highly sialylated variant is expected. In other words, a slower gradient rate may demand a slower flow rate and therefore the expected variant may elute at a later time point.

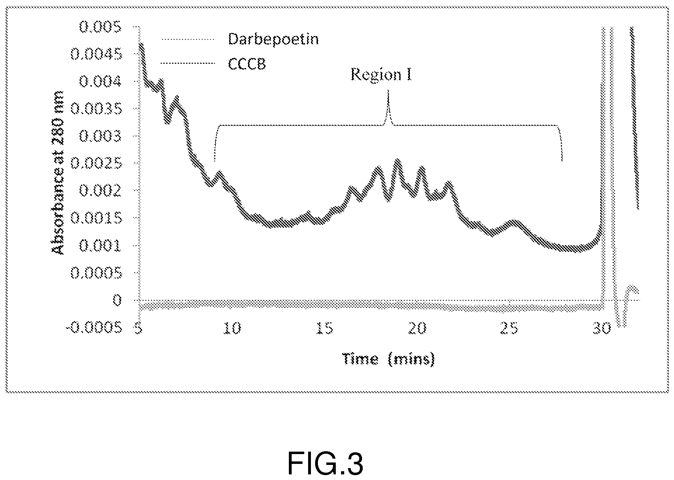

Fig. 3 is magnification of the Region I of Fig. 1, and illustrates the importance of the pH gradient. Use of the gradient enabled fractionation and elution of any low sialylated variant/s of the protein that bound to the column. Whereas, use of a salt concentration of about 90 mM led at the equilibration step lead to diminished binding of low sialylated variants of the protein to the column, and elution with a pH gradient at similar salt concentration led to preemptive elution of low sialylated variants of the protein, thus enriching the highly sialylated variant/s.

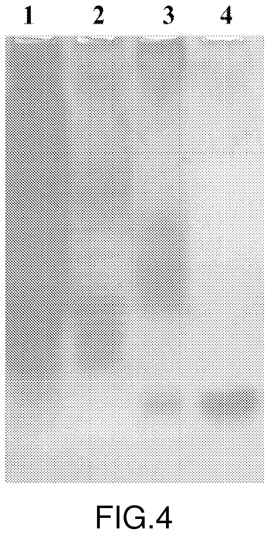

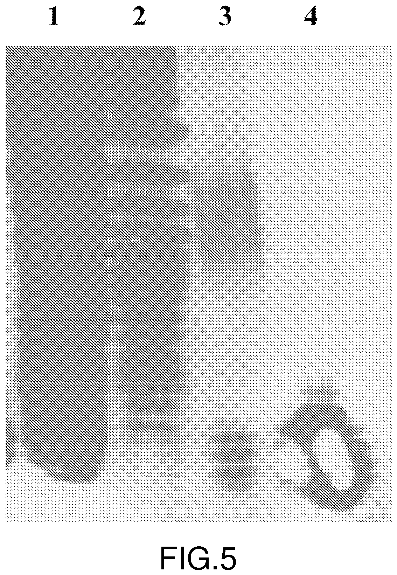

Isoelectric focusing and western blot analysis (Fig. 4 and 5 respectively) was used to determine enrichment of highly sialylated variant of darbepoetin. Lane 1 and 2 represent the CCCB and flow-through from the column respectively. Lane 3 represents the enriched highly sialylated variant obtained post elution using the pH gradient at constant salt concentration. Lane 4 represents darbepoetin used as control.

## Claims

1. A method for fractionation of highly sialylated variants of darbepoetin comprising:
a) loading a clarified cell culture broth comprising a mixture of differentially sialylated darbepoetin on to a strong anion exchange resin, and
b) eluting the bound protein using a pH gradient at a constant salt concentration, wherein the eluate is enriched in highly sialylated variants of the protein.

2. A method according to claim 1, wherein the strong anion exchange resin is pre-equilibrated with a buffer comprising 90 mM sodium chloride and wherein the pH of the equilibration buffer is between the isoelectric point of the sialylated variant and the neutral pH.

3. A method according to claim 1, wherein the pH gradient is a linear pH gradient established between about 7.5 and about 2.0.

4. A method according to claim 1, wherein the said method is a single step fractionation method to isolate highly sialylated variants of darbepoetin and is devoid of any in process wash step.

5. A method according to claim 1, wherein the conductivity of the elution buffer is equal to that of the equilibration buffer.

6. A method according to claim 1, wherein highly sialylated variants of darbepoetin contains at least about 18 or more sialic acid moieties attached to the protein.

## Patentansprüche

1. Verfahren zum Fraktionieren von hoch sialylierten Varianten von Darbepoetin, das Folgendes beinhaltet:
a) Laden einer geklärten Zellkulturbrühe, die ein Gemisch von differential sialyliertem Darbepoetin umfasst, auf ein starkes Anionenaustauschharz, und
b) Eluieren des gebundenen Proteins mit einem pH-Gradienten bei einer konstanten Salzkonzentration, wobei das Eluat in hoch sialylierten Varianten des Proteins angereichert ist.

2. Verfahren nach Anspruch 1, wobei das starke Anionenaustauschharz mit einem Puffer voräquilibriert wird, das 90 mM Natriumchlorid umfasst, und wobei der pH-Wert des Äquilibrierungspuffers zwischen dem isoelektrischen Punkt der sialylierten Variante und dem neutralen pH-Wert liegt.

3. Verfahren nach Anspruch 1, wobei der pH-Gradient ein linearer pH-Gradient ist, der zwischen etwa 7,5 und etwa 2,0 gebildet wurde.

4. Verfahren nach Anspruch 1, wobei das genannte Verfahren ein einstufiges Fraktionierungsverfahren zum Isolieren von hoch sialylierten Varianten von Darbepoetin und ohne jeden Prozesswaschschritt ist.

5. Verfahren nach Anspruch 1, wobei die Leitfähigkeit des Elutionspuffers gleich der des Äquilibrierungspuffers ist.

6. Verfahren nach Anspruch 1, wobei hoch sialylierte Varianten von Darbepoetin wenigstens etwa 18 oder mehr an das Protein angelagerte Sialsäurebestandteile enthalten.

## Revendications

1. Procédé de fractionnement de variantes fortement syalilées de darbépoétine comprenant :
a) le chargement d'un bouillon de culture de cellules clarifiées comprenant un mélange de darbépoétine sialylée sur une résine à fort échange d'anions, et
b) l'élution de la protéine liée à l'aide d'un gradient pH à une concentration de sel constante, dans lequel l'éluat est enrichi en variante fortement sialylées de la protéine.

2. Procédé selon la revendication 1, dans lequel la résine à fort échange d'anions est prééquilibrée avec un tampon comprenant 90 mM de chlorure de sodium et dans lequel le pH du tampon d'équilibrage est compris entre le point isoélectrique de la variante sialylée et le pH neutre.

3. Procédé selon la revendication 1, dans lequel le gradient de pH est un gradient de pH linéaire établi entre environ 7,5 et environ 2,0.

4. Procédé selon la revendication 1, ledit procédé étant un procédé de fractionnement en une seule étape pour isoler des variantes fortement sialylées de darbépoétine et dépourvu de toute étape de lavage en cours de traitement.

5. Procédé selon la revendication 1, dans lequel la conductivité du tampon d'élution est égale à celle du tampon d'équilibrage.

6. Procédé selon la revendication 1, dans lequel des variantes fortement sialylées de darbépoétine contiennent au moins environ 18 ou plus de fractions d'acide sialique attachées à la protéine.
